# EUROPEAN PATENT APPLICATION

(11) **EP 2 596 754 A1**
(43) Date of publication of application: **29.05.2013**
(21) Application number: 11190424.9
(22) Date of filing: 23.11.2011
(51) Int. Cl.: A61B 17/00

(54) **Medical implant and manufacturing method thereof**

(71) Applicant: Occlutech Holding AG, 8201 Schaffhausen (CH)
(72) Inventor: Ottma, Rüdiger, 99441 Großschwabhausen (DE); Nielsen, Stevan, 72108 Rottenburg Am Neckar (DE)
(74) Representative: KIPA AB

(57) **Abstract**

A medical implantable occlusion device (100-800) is disclosed comprising a braiding of at least one thread, said braiding comprising an expanded diameter portion (102) at a distal end of the device, a tubular member (103) at a proximal end of the device extending along a longitudinal axis (104). The expanded diameter portion comprises loops (1103, 1104, 1204, 1304), formed by loop strands (1105, 1106, 1206, 1306) of the at least one thread, returning to the proximal end. An apex point (1107, 1108, 1208, 1308) of each of the loop strands corresponds to the turning point of the loop strands and to the point of each of the loop strands being arranged closest to a centre point (1117) of the expanded diameter portion, and the apex points are positioned intermediate between the centre point (1117) and a periphery (1113) of the expanded diameter portion.

## Description

### Field of the Invention

This invention pertains in general to the field of medical implants, as well as methods for manufacturing such implants. More particularly the invention relates to an intraluminally deliverable occlusion device for selective occlusion of a target site in a body lumen, such as the body's circulatory system.

### Background of the Invention

Various intravascular deliverable devices are used for treating specific conditions via access through body lumina, such as patients circulatory system. The target site may for instance be an atrial or ventricular septum having a defective opening to be occluded, such as devices for treating septal defects and the like. In certain circumstances, it may be necessary to occlude a patient's lumen, vessel, chamber, channel, hole, or cavity such as to stop blood flow there through. One such condition known in the art is a patent ductus arteriosus (PDA), which is essentially a condition wherein two blood vessels, most commonly the aorta and pulmonary artery adjacent the heart, have a blood flow shunt between their lumens. Blood can flow directly between these two blood vessels through the passageway, compromising the normal flow of blood through the patient's vessels. Other physiologic conditions in the body occur where it is also desirous to occlude a vessel, a shunt between vessels, or an ostium at a branch vessel, in order to prevent blood flow through the vessel.

An occlusion device for treatment of such heart disease is disclosed in United States patent application publication number 2009/0187214. A tubular member of a braided fabric is disclosed having disc-shaped portions connected to either side of the tubular member via transition segments having reduced cross-sectional dimension. The device is delivered by a catheter connected to either of the discs. The discs engage with the walls of the septum. The diameter and length of the PDA is determined by angiography in order to select the size of the device, where the latter dimension requirement is a particular issue due to the double disc design, which furthermore is increasing the complexity of the device and the delivery thereof.

EP2014240 discloses a medical device having a cylindrical body portion, a disc portion, and a small transition diameter there between. The transition diameter is recessed within an indention. A problem with the device is lack of flexibility and compactness of the device.

A problem with prior art is to achieve sufficient flexibility while maintaining a compact device. This applies in particular during delivery through tortuous channels. Flexibility is also desired during deliver when releasing the implantable device out of a catheter to the target site. Moreover, a degree of flexibility to accommodate anatomical movements without fatigue or risk of loosening from the implantation site during an ingrowth period is also a desired characteristic of such devices. Further, a compact device is desirable for quick and easy delivery, for example via a catheter, and for occupying less space in the body, thereby reducing chance of interference with bodily functions. A more flexible implant is desired in terms of adjustments of the device used and applied methods, due to the fact that different patients are anatomically slightly different to one another and that the deficiency to be treated is most often individual and unique for each patient treated.

The issue with insufficient flexibility of prior art devices is further discussed in the following. Issues with some prior art solutions are that the braided devices are not sufficiently flexible, and/or that a large force is required to manipulate the device, for example due to too high stiffness of the braided mesh of the device. This may lead to a difficult delivery of the braided device through for example a catheter. For braided devices having an expanded and a collapsed shape configuration the large force needed to collapse the device from the relaxed expanded state may lead to difficulties to pull the device into for example the delivery sheath of the catheter. Also, due to this force, making these braided device less flexible, the friction between the device and the catheter will be too high in order to easily move and manipulate the device in the catheter, for both movement to pull and to push the device in the catheter. Thus, there is a need for a braided device which allows a secure deployment in the patient.

Insufficient flexibility of some braided devices known in the art may also make the positioning of the device in the patient's body more difficult, for example, by the inability for the device to adapt to the unique anatomy of the target site as mentioned above. Further, a stiff device may lead to injury at the target site, for example to soft tissues in contact with the device. There is accordingly a need for a braided device which adjusts for differences in the anatomies between patients. Further an inflexible device may cause embolies, which could be transported to organs such as the brain and cause blood clots. This appears in particular to be the case with some devices having ends clamped together. In particular it may be an issue to have a distal end having a structure protruding into an arterial (high blood pressure) blood stream leading to vital organs, such as the brain. One issue are protruding threaded clamps keeping together a bundle of strands, such as described in WO99/12478.

W02008/040555 discloses a braided occlusion device having folded sections in two or more layers for positioning in an opening. Sections at the distal portion of the device are backbent towards the proximal portion to contact the tissue of the wall having the opening to be occluded. The folded sections cause the device to exhibit a substantial amount of wires to be deformed when compressing the device, hence increasing the force necessary to compress the device and the cross-section of the compressed device.

US2005/0283962 discloses a method of manufacturing a device of a tubular braiding. An issue with tubular braidings as disclosed in US2005/0283962 is insufficient stability that may lead to dislocation of the device from the implanted site.

An issue with prior art braided devices is that catheters with too large cross-section are required, as the devices take up large space even when in the collapsed state. Some regions of the body may thus only be reached with difficulty by such devices requiring large diameter catheters.

A further disadvantage with prior art is that some devices designed to be flexible may not have the sufficient retention force to withstand external forces.

A further problem with prior art is to achieve a secure attachment of the device in the body while maintaining ease of delivery. Secure attachment is necessary for patient safety and achieving the intended treatment of the septal defect.

Thus, there is a need for an implant which adjusts for differences in the cardiovascular system between patients still allowing a secure deployment of a medical implant.

Also, a problem with prior art is that delivery of the device via the aorta having a high pressurized blood environment may give rise to several undesired conditions. Debris, e.g. from arteriosclerotic deposits scraped loose by the device under delivery, for instance at ostia of branch vessels, may cause embolic complications. Embolic material could thus be transported to vital organs, e.g. to the brain via the subclavian vessels, and cause blood clots, leading to stroke.

The above problems may have dire consequences for the patient and the health care system. Patient risk is increased.

Hence, an improved implant would be advantageous and in particular allowing for increased flexibility, cost-effectiveness, and/or patient safety would be advantageous. Also, and a method for manufacturing such medical implant would be advantageous.

### Summary of the Invention

Accordingly, embodiments of the present invention preferably seeks to mitigate, alleviate or eliminate one or more deficiencies, disadvantages or issues in the art, such as the above-identified, singly or in any combination by providing a device and a method according to the appended patent claims.

Embodiments of the present invention may be well suited for the selective occlusion of a vessel, lumen, channel, hole, cavity, or the like. One particular example, without limitation, of such a condition is a Patent Ductus Arteriosus (hereinafter PDA). Another example is a vessel, lumen, channel, hole or shunt, through which blood flows from one vessel to another vessel such as an Atrial Septal Defect (herein after ASD) or a Ventricular Septal Defect (herein after VSD). Other examples could be an Arterial Venous Fistula (AVF), Arterial Venous Malformation (AVM), a Patent Foramen Ovale (PFO), or a Para-Valvular Leak (PVL).

According to a first aspect of the invention a medical implantable occlusion device is provided comprising a braiding of at least one thread, said braiding comprising an expanded diameter portion at a distal end of the device, and a tubular member at a proximal end of the device extending along a longitudinal axis. The expanded diameter portion comprises loops, formed by loop strands of the at least one thread, returning to the proximal end. An apex point of each of the loop strands corresponds to the turning point of the loop strands and to the point of each of the loop strands being arranged closest to a centre point of the expanded diameter portion, and the apex points are positioned intermediate between the centre point and a periphery of the expanded diameter portion.

According to a second aspect of the invention a method of manufacturing a medical implantable occlusion device of a braiding of at least one thread is provided. The method comprises forming loop strands of said at least one thread at a distal end of said device, returning to said proximal end, whereby an apex point of each of said loop strands corresponds to the turning point of the loop strands and to the point of each of the loop strands being arranged closest to a centre point of the distal end, and forming the braiding in a first heat setting step to a first shape comprising a tubular member at the proximal end and an expanded diameter portion at the distal end, wherein the apex points are positioned intermediate between a centre point of the expanded diameter portion and a periphery of the expanded diameter portion.

Further embodiments of the invention are defined in the dependent claims, wherein features for the second and subsequent aspects of the invention are as for the first aspect mutatis mutandis.

Some embodiments of the invention provide for a flexible braided medical device that is easy to manipulate in a delivery device and that adapt to varying anatomical sites in a body of a human or animal.

Some embodiments of the invention also provide for secure attachment of a medical implant in a patient's vascular system.

Some embodiments of the invention provide for a compact medical implant with maintained flexibility.

Some embodiments of the invention provide for a medical implant that can be safely delivered to a treatment site in a patient.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is an illustration of a medical implantable occlusion device according to an embodiment of the invention;
Fig. 2 is an illustration of a medical implantable occlusion device according to an embodiment of the invention;
Fig. 3 is a side view the medical implantable occlusion device in Fig. 2;
Figs. 4a-c are illustrations of a medical implantable occlusion device according to an embodiment of the invention during a manufacturing step;
Fig. 5 is a flow chart illustrating a method of manufacturing of a medical implantable occlusion device according to an embodiment of the invention; and
Fig. 6 is a flow chart illustrating a method of occluding a shunt in a body lumen with a medical implantable occlusion device according to an embodiment of the invention.
Fig. 7 is an illustration of a medical implantable occlusion device according to an embodiment of the invention;
Fig. 8 is a top view the medical implantable occlusion device in Fig. 7;
Fig. 9 is a perspective view the medical implantable occlusion device in Fig. 7;
Fig. 10 is an illustration of a top view of the medical implantable occlusion device according to an embodiment of the invention;
Fig. 11 is an illustration of a top view of the medical implantable occlusion device according to an embodiment of the invention;
Fig. 12 is an illustration of a top view of the medical implantable occlusion device according to an embodiment of the invention;
Fig. 13 is an illustration of a top view of the medical implantable occlusion device according to an embodiment of the invention;
Fig. 14a-b are illustrations of a medical implantable occlusion device according to an embodiment of the invention;
Fig. 15a-b are illustrations of a medical implantable occlusion device according to an embodiment of the invention;
Figs. 16a-b are illustrations of a method for manufacturing of an implantable occlusion device according to an embodiment of the invention;
Fig. 17 shows an implantable occlusion device according to an embodiment of the invention;
Fig. 18 shows a method of manufacturing a medical implantable occlusion device according to an embodiment of the invention;
Fig. 19 a flow chart illustrating a method of manufacturing of a medical implantable occlusion device according to an embodiment of the invention; and

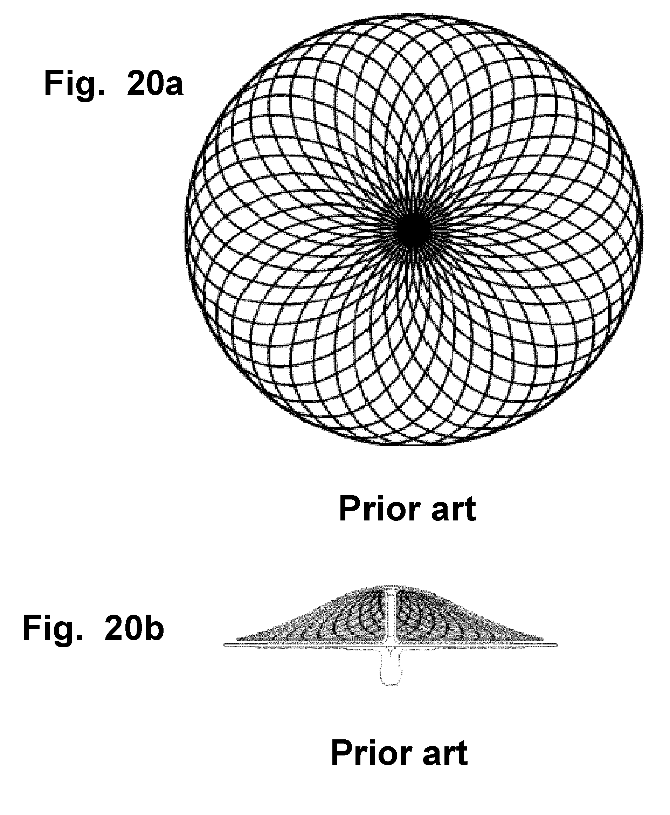

Figs. 20a-b are illustrations of prior art devices.

### Description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on embodiments of the present invention applicable to PDA plugs. Some embodiments also relate to PFO or ASD devices. However, it will be appreciated that the invention is not limited to these application but may be applied to many other medical implantable devices, including for example filters, stents, Left Atrial Appendage (LAA) occluders, aneurysm treatment devices, grafts, etc.

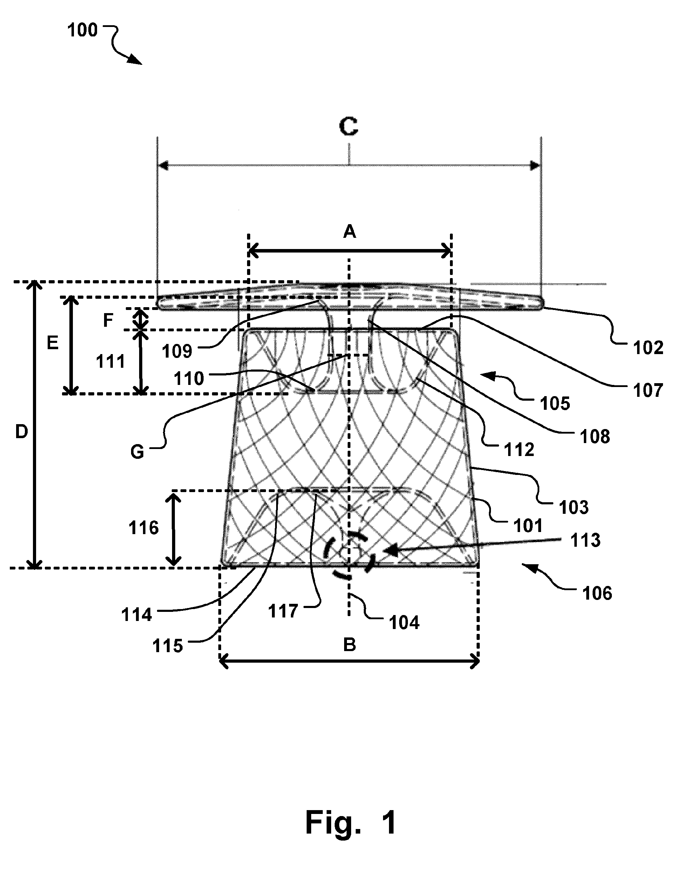

Fig. 1 shows a medical implantable occlusion device 100 according to an embodiment of the invention. The device 100 comprises a mesh or braiding 101 of at least one thread. The braiding 101 may be formed from one thread or several. The device 100, or more particularly the braiding 101, has an unloaded relaxed state and a stretched state. Thus, in the relaxed state, wherein the device 100 has a shape as depicted in Fig. 1, no external force acts on the device 100. The device 100 may be stretched and thereby exhibit a smaller cross-section, in order to fit inside a delivery device such as a catheter. The device 100 may be self-expandable between the stretched state and the relaxed state, i.e. when the device 100 is removed from the confinement of the catheter the cross-section of the device 100 returns to its originally defined value in the unloaded relaxed state. The device may be self-expandable due to an inherent elasticity of the threads in the braiding. The device may also have a shape memory, e.g. triggerable to go to the relaxed state at a switching temperature, such as body temperature. Alternatively, or in addition, in other embodiments of the implantable device, expansion devices (not shown), such as inflatable balloons, may be used to bring the device from the collapsed state to the expanded, relaxed state.

The shape of the device 100 in the relaxed state may be defined in a heat treatment procedure of the device 100 or more particularly of the braiding 101. The dimensions of the device 100 in the relaxed state are defined in the heat treatment procedure if the braiding. A particular embodiment of the manufacturing method is described below.

The entire device 100 may be comprised of a single, continuous braiding 101. The braiding 101 may be made of a material suitable for implanting in a human or animal body, and suitable for being formed in a heat treatment procedure to a desired shape in the unloaded relaxed state and also in the stretched state. For example NiTinol may be used as a material for the device 100. However, suitable materials for embodiments of the braiding are various and include shape memory materials, metal, superelastic alloys (such as NiTinol), or polymers, such as degradable polymers.

The braiding 101 comprises an expanded diameter portion 102, and a tubular member 103 extending along a longitudinal axis 104. The tubular member 103 has a distal region 105 towards the expanded diameter portion 102 and an opposite proximal region 106. The distal region 105 comprises a distal rim 107 corresponding to the distal endpoint of the distal region 105 closest to the expanded diameter portion 102. The distal rim 107 corresponds to the edge of the tubular member 103 that faces the expanded diameter portion 102, and is aligned in a plane substantially orthogonal to the longitudinal axis 104.

The braiding 101 comprises a stem 108 having a first end 109 connected to the expanded diameter portion 102 and a second end 110 connected to the distal region 105 of the tubular member 103. In the unloaded relaxed state of the device 101 as shown in Fig. 1 the stem 108 is partly sunk into the tubular member 103 beneath the distal rim 107. Thus, the second end 110 of the stem 108 is displaced from the distal rim 107 by a first offset 111 along the longitudinal axis 104.

The displacement of the stem 108 into the tubular member 103 provides a more compact device 100. As the length (E) of the stem 108 is maintained while the device 100 is more compact, i.e. due to decreased length (D), the function of the stem 108 for providing flexibility between the tubular member 103 and the expanded diameter portion 102 is maintained.

By stretching the device 100, e.g. by moving the expanded diameter portion 102 and the tubular member 103 in opposite directions along the longitudinal axis 104, the device 100 is put in the stretched state and the first offset 111 may decrease. As the stem 108 is partially sunk into the tubular member 103 the amount of displacement possible of the device 100 from the unloaded relaxed state, e.g. by aforementioned stretching, could be increase as the stem 108 may be stretched out of the tubular member 103, for example by an amount corresponding to the offset 111 of the device in the relaxed state. Various offsets 111 may be defined for the device 100 in the relaxed state.

Alternatively, or in addition, the length of the stem 108 may substantially remain constant without being stretched. This allows for a particular stable long-term construction even in anatomical situations where a continuous movement at the implantation site is present. The stem may be made of a portion of parallel threads or a more densely braided section of the braiding 101 at the stem 108, providing for particular strength in the longitudinal direction.

The distal region 105 has a curved braiding having an apex at the second end 110 of the stem and a distal surface 112. This may provide an inherent flexibility in use, drawing the tubular part 103 and the expanded diameter part 102 longitudinally towards each other. The shape and spatial extent of the distal surface 112 provides a spring like portion. This spring-like portion is shown left and right to the diameter G in Figure 1. This provides for advantageously controllable self-contraction properties of the device 100. As substantially the entire distal surface 112 enclosed by the distal rim 107 is recessed, as seen in Fig. 1, the range of motion and flexibility or spring-like action is improved, as discussed further below. I.e. the diameter of the recessed distal surface 112 may be substantially equal to the diameter of the distal rim 107. Also the distal surface 112 may be substantially concave towards the expanded diameter portion 102, as seen in Fig. 1, and/or having a portion close to the second end 110 extending radially substantially parallel to the expanded diameter portion 102. This further allows for increased flexibility of the stem 108 and expanded portion 102 as the area of the flexible recessed surface 112 is increased, as the amount of material being flexible is increased, without increasing the dimensions of the device 100 externally. As the device 100 may strive towards the relaxed state by being self-expandable, or rather self-contracting, between the stretched state and the relaxed state, an inherent force may act on the stem 108 to maintain the offset 111 as defined in the relaxed state. By removing the external forces on the device 100 and thereby release it from the stretched state, the braiding 101 may return to its original shape in the relaxed state due to the aforementioned inherent force. The original shape of the device 100 may be defined in a heat treatment procedure in a manufacturing step. The inherent force pulls the expanded diameter portion 102 towards the tubular member 103 as soon the stem 108 is displaced from the shape of the device 100 in the relaxed state. As the stem 108 is partially sunk into the tubular member 103 a large flexibility is obtained as the device 100 may be stretched by expansion of the stem 108 from the tubular member 103 with reduction of the offset 111 from its defined value in the relaxed state while the inherent force act to contract the expanded diameter portion 102 towards the tubular member 103. This is advantageous as a single device 100 can be applied to occlude various PDA with different lengths while maintaining secure attachment of the device 100 at the PDA as the diameter portion 102 will be contracted towards the tubular member 103 for each length configuration possible for the device 100, i.e. for each displacement of the stem 108 relative to the distal rim 107.

The tubular member 103 may comprise biocompatible fibres or patches of for example of PET that support sealing of the blood flow through the device 100, and thereby the shunt to be occluded.

The tubular member 103 may be tapered towards the expanded diameter portion 102. The diameter (B) of the endpoint of the proximal region 106 is thereby larger than the diameter (A) of the endpoint of the distal region 105. When positioned in the PDA the tapered shape towards the expanded diameter portion 102 create an increased force acting on the walls of the PDA to be occluded as the expanded diameter portion 102 is pulled towards the tubular member 102 by the aforementioned inherent force if the device 100 is self-contracting. The proximal region 106 will exert a compressive force towards the expanded diameter portion 102 by the tapered configuration in comparison to a pure frictional force in case the device is not tapered, or tapered in the other direction. The tapered shape, similar to a champagne cork, has a function similar to an oversized cork at one end, leading to improved retention in body lumina. This is in particular advantageous with shunts that anatomically have an increasing lumen diameter in the same direction as the tapering and where the expanded diameter portion provides further increases locking of the device in the shunt. In particular the expanded diameter portion is substantially flat and has a diameter larger than an ostium of the shunt into which it is placed. Thus the rim of the periphery of the expanded diameter portion may advantageously engage the tissue around the ostium. The closure effect is further improved when a higher pressure is applied from the distal side towards the distal end of the device, such as when the device 100 is arranged in a PDA. Here, the tubular portion is arranged in the shunt channel of the PDA and the expanded diameter portion is arranged in the aortic arch around the ostium of the PDA. Thus a secure occlusion is achieved even before the device is securely covered with endothelia and tissue integrated with the surrounding tissue.

The distance (F) between the distal rim 107 and the expanded diameter portion 102 may be substantially smaller than the length of the stem 108. By increasing the offset 111 in relation to the distance (F) a more compact device 100 may be obtained while maintaining the flexibility discussed above. The distance (F) may be increased so that the expanded diameter portion 102 and the stem 108 may pivot more from side to side without the expanded diameter portion 102 interfering with the distal rim 107.

The stem 108 may be pivotable with respect to the tubular member 103 around the second end 110 of the stem 108. In this way the angle of the expanded diameter portion 102 in relation to the tubular member 103 may be changed depending on the anatomy of the site in the vascular system to be occluded. The length (F) of the stem 108, and the amount of offset 111 and distance (F) may affect the amount the stem 108 may be pivoted and thereby the amount of angular displacement of the expanded diameter portion 102.

The stem 108 may be concentric with respect to the tubular member 103. A symmetric shape of the device 100 may increase the flexibility of the expanded diameter portion 102 in relation to the tubular member 103. An asymmetric configuration may be suitable in particular anatomies to be occluded.

ln Fig. 1 the second end 110 of the stem 108 is connected to the distal surface 112 of the distal region 105. The distal surface 112 is sunk into the tubular member 103 beneath the distal rim 107. As the second end 110 of the stem 108 is connected to the distal surface 112 which is sunk into the tubular member 103 beneath the distal rim 107 the flexibility of the stem 108 in relation to the tubular member 103 may be increased. The entire distal surface 112 may be flexible to allow movement of the stem 108 in relation to the tubular member 103. The stem 108 may be flexible by itself as the braiding 101 forming the stem 108 may provide for flexibility. Alternatively, or in addition, the stem 108 may be of a flexible material. Tilting of the distal surface 110 in relation to the longitudinal axis 104 may provide for increased angular displacement of the expanded diameter portion 102 in relation to the tubular member 103.

The distal surface 112 may be confined to a region of the braiding 101 between the stem 108 and the distal rim 107. The diameter of the distal surface 112 may be substantially equal to the diameter of the distal rim 107, i.e. substantially equal to the diameter (A) in Fig. 1. Hence, the surface of the braiding 101 inside the distal ring 107 is sunk into the tubular member 103. As a larger portion of the surface of the braiding between the second end 110 of the stem 108 and the distal rim 107 is sunk into the tubular member 103 an increased range of motion may be possible for the stem 108, and thereby an increased flexibility of the expanded diameter portion 102. This is in contrast to cases where only a minor part of the distal surface is recessed, which could not provide the same range of motion of the distal surface 112 and thereby not the same range of motion of the expanded diameter portion 102. Hence, in such cases, the device will not be able to conform to such wide range of anatomies as the device 100 of the current invention.

The expanded diameter portion 102 may be a disc shaped portion. The disc may be bent with its outer periphery closer towards the tubular portion than the center portion thereof. Alternatively, it may have any shape suitable for the particular anatomy of the site to be occluded.

The outer diameter (C) of the expanded diameter portion 102 may be substantially larger than a diameter of the tubular member 103, as depicted in Fig. 1. An increased diameter (C) may reduce the pressure exerted by the expanded diameter portion 102 on the vessel wall at the occlusion site, and decrease the risk of the device 100 being dislodged from the occlusion site. The diameter may be equivalent to the largest cross-section throughout the disclosure.

The diameter (G) of the stem 108 may be substantially smaller than any diameter of the tubular member (A, B). A smaller diameter may increase the flexibility of the expanded diameter portion 102.

The proximal portion 102 may comprise a connecting member 113 for a delivery device (not shown). The delivery device may grasp the connection member 113 which may be spherical in shape, thus providing a pivoting motion of the device 100 in relation to the delivery device in combination with secure attachment. The ends of the at least one thread forming the braiding 101 may be fixed to the connecting member 113. The connecting member 113 may thus be a weld or any other attachment means for the threads of the braiding 101. The connecting member may comprise a threaded screw attachment (not shown) of female or male type for threaded attachment to a delivery device having corresponding threads. The expanded diameter portion 102 may comprise returning loops of the at least one thread, meaning that opposite ends of the at least one thread forming the expanded diameter portion 102 are fixed to the connecting member 113. By having returning loops only one collection point for the ends of the at least one thread is needed. The connection member 113 may thus serve as a connection for these ends, thereby avoiding multiple connection points such as welds on the expanded diameter portion 102. Hence, a flat expanded diameter portion 102 may be provided, that increases the compactness of the device 100. Thereby no parts of the device 100 extend beyond the expanded diameter portion. By having such compact device 100, it may be easily positioned and manipulated at the target site. This is in contrast to cases having protruding parts at both the distal and proximal ends, which could not attain the same degree of compactness of the device. Further, due to the connection member 113 on the proximal end 106, the device 100 may be delivered through the vena cava with improved safety to the patient. Delivery to the high pressure arterial side of the vascular system is avoided, which provides for less complications and a medical procedure which is simpler to perform.

Similarly to the distal region 105 of the device 100 the proximal region 106 may have a proximal rim 114 and a proximal surface 115. A distal end 117 of the connection member 113 may have a second offset 116 in relation to the proximal rim 114.

The proximal surface 115 may be confined to a region of the braiding 101 between the connecting member 113 and the proximal rim 114. The diameter of the proximal surface 115 may be substantially equal to the diameter of the proximal rim 114, i.e. substantially equal to the diameter (B) in Fig. 1. Hence, the surface of the braiding 101 inside the proximal ring 114 is sunk into the tubular member 103. This may provide increased flexibility of the connecting member 113 in relation to the tubular member 103, and also the device 100 becomes more compact, as the entire length of the connecting member 108 may be sunk into the tubular member 103. Alternatively, in other embodiments, the connecting member 113 may protrude proximally of the tubular portion.

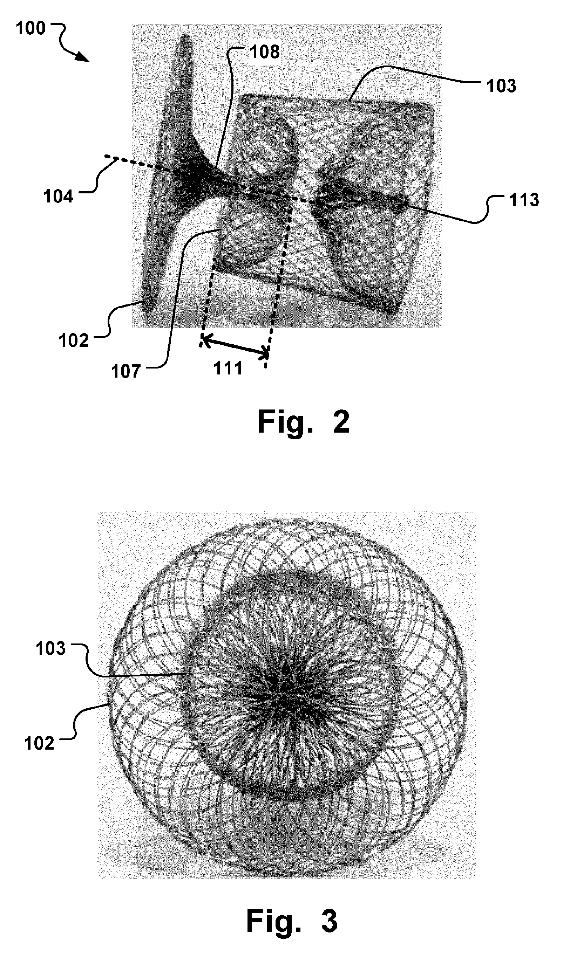

Fig. 2 shows a perspective view of a medical implantable occlusion device 100 according to an embodiment of the invention. The stem 108 of the device 100 has an offset 111 from the distal rim 107 into the tubular member 103, providing the advantages as described above. A connecting member 113 in the form of a spherical ball is provided at the proximal end for connecting to a mating socket.

Fig. 3 shows a side view of the device 100 in Fig. 1. The expanded diameter portion 102 is disc-shaped and is substantially concentrically aligned with the tubular member 103.

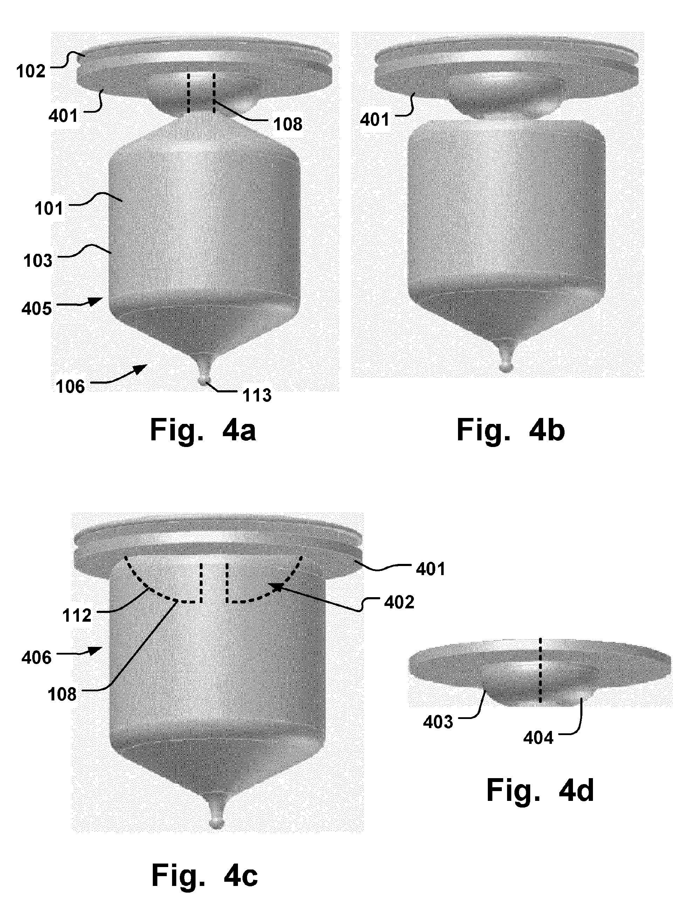

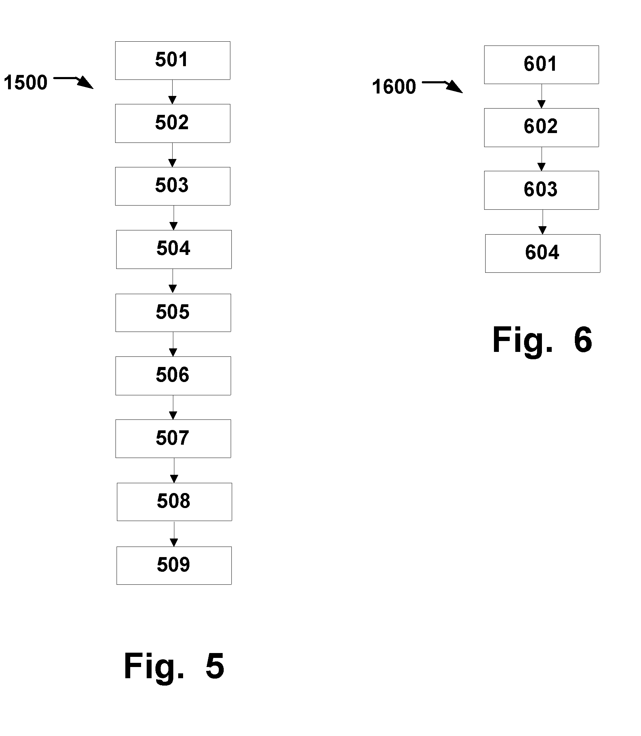

Fig. 4 and Fig. 5 illustrate a method 1500 of manufacturing a medical implantable occlusion device 100-800, of a braiding 101 of at least one thread. The method comprises forming 501 the braiding 101 in a first heat setting step to a first shape 405 comprising a tubular member 103 and an expanded diameter portion 102. A secondary tool 401 is attached 504 to the braiding 101 having the first shape 405. The braiding 101 is in the following formed 506 in a second heat setting step to a second shape 406 by the secondary tool 401, as shown in Figs. 4b-c. The braiding 101 maintains partly the first shape 405.

The method 1500 comprises further welding 503 a bundle of threads of the braiding 101 at a proximal end 106 of the tubular member 103 after forming the first shape 405. Alternatively, the welding may be performed after any manufacturing step. The method 1500 comprises annealing 502, 509, the braiding 101 in the heat setting steps after forming each of the first and second shapes 405, 406, respectively. By annealing the braiding 101 the desired shapes 405, 406, can be maintained.

When forming the second shape 406 the method 1500 comprises moving 507 the secondary tool 401 into the braiding 101 to produce at least one recess 402 into the tubular member 103. ln Fig. 4b the secondary tool 401 is partly moved towards tubular member 103, and in Fig. 4c the secondary tool 401 is sunk into the tubular member 103 creating a recess 402. Similarly, a secondary tool may be sunk into the proximal region such that the connecting member 113 is sunk into the tubular body 103.

ln forming the second shape 406 the method 1500 comprises attaching 505 the secondary tool around the stem 108 of the braiding 101. The stem 108 is between and connects the tubular member 103 and the expanded diameter portion 102. When moving 508 the secondary tool 401 into the tubular member the stem 108 is partly sunk into the tubular member 103 as illustrated in Fig. 4c. The braiding 101 in the second shape 406 with the stem 108 recessed into the tubular member 103 may then be annealed and the secondary tool 401 may be removed. The second shape 406 may then correspond to the unloaded relaxed state of the braiding 101 of the device 100-800. In other embodiments the secondary tool 401 may be attached to the braiding 101 at any location between the proximal region 106 and the opposite end point of the device 100-800, such as the expanded diameter portion 102, for forming the device 100-800, into a second shape 406 that may be integral with the first shape 405, e.g. the first and second shapes 405, 406 may define the outside and inside contours of the device 100-800, respectively.

The secondary tool may comprise at least two connectable portions 403, 404 for attachment around the stem 108 of the braiding 108.

Fig. 6 illustrates a medical method 1600 of occluding a shunt in a body lumen, comprising providing 601 a device 100-800, inserting 602 the device 100-800 in a collapsed state into the shunt, expanding 603 and releasing the device 100-800, in the shunt, thus anchoring 604 the device 100-800, in the shunt for occluding the latter by the device 100-800.

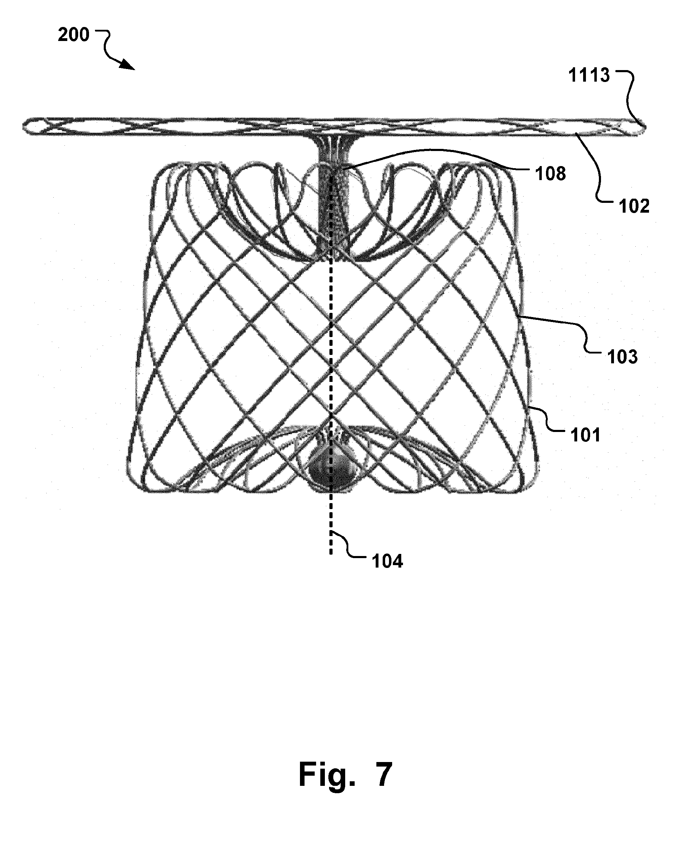

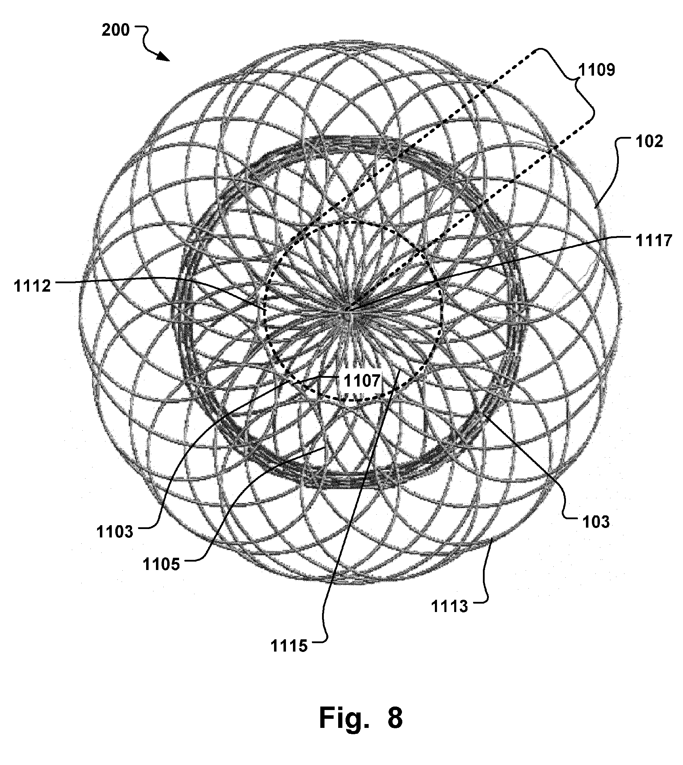

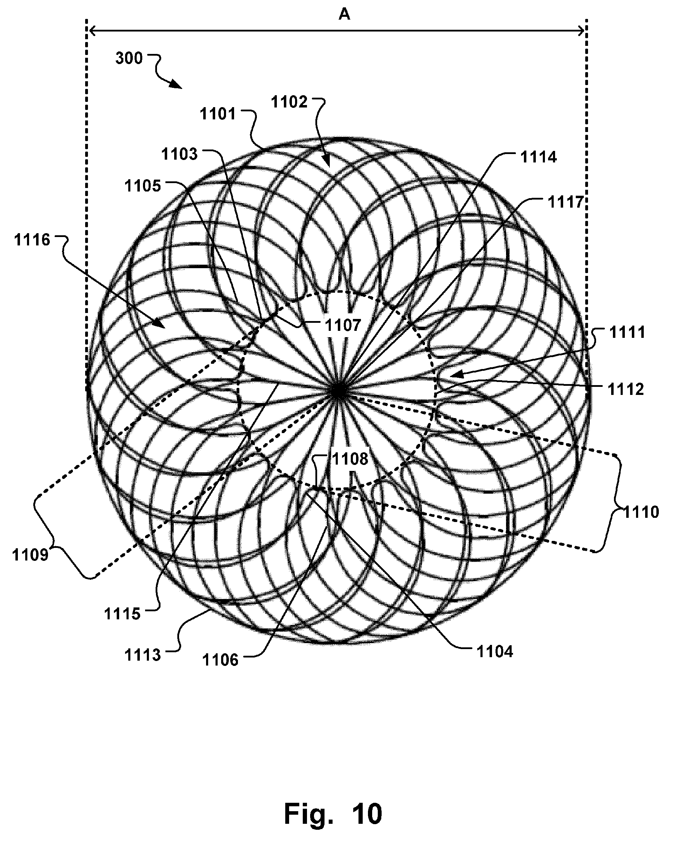

Fig. 7 illustrates a device 200 according to another embodiment of the invention. It has an expanded diameter portion 102 at a distal end of said device, a tubular member 103 at a proximal end of said device extending along a longitudinal axis 104. The expanded diameter portion is shown in more detail in Fig. 8 in a top-view, and it comprises loops 1103 (1104, 1204, 1304, cf. Figs. 10-12), formed by loop strands 1105 (1106, 1206, 1306, cf. Figs. 10-12) of said the least one thread, that returns to the proximal end. Apex points 1107 (1108, 1208, 1308, cf. Figs. 10-12) of each of the loop strands corresponds to the turning point of the loop strands and to the point of each of the loop strands being arranged closest to a centre point 1117 of the expanded diameter portion. The apex points are positioned intermediate between the centre point 1117 and a periphery 1113 of the expanded diameter portion. This way less wires will cross the centre 1117 of the expanded diameter portion and the flexibility of the expanded diameter portion will increase so that it can conform freely to different anatomies with less retention force. The braiding of the expanded diameter portion is discussed in more detail below, and with reference to Figs. 10-13.

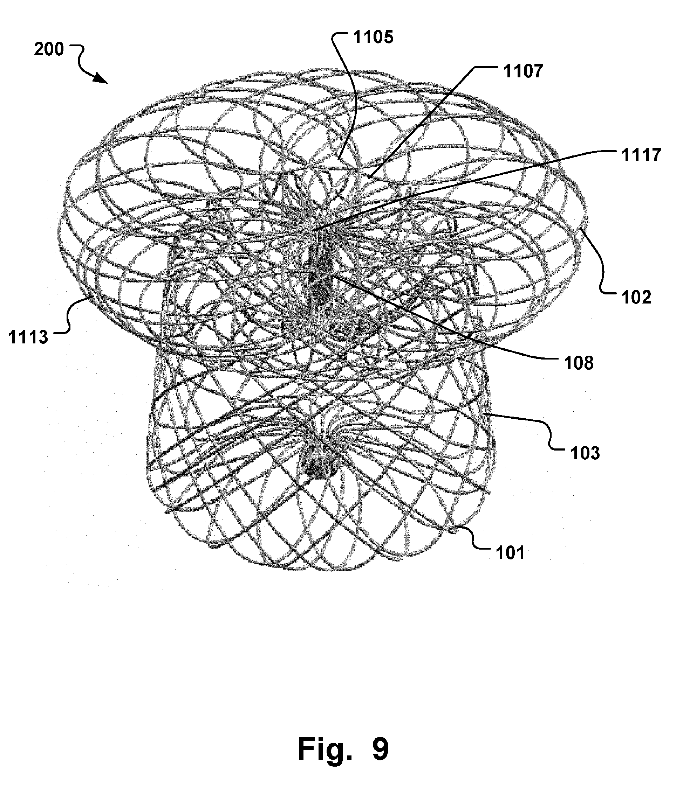

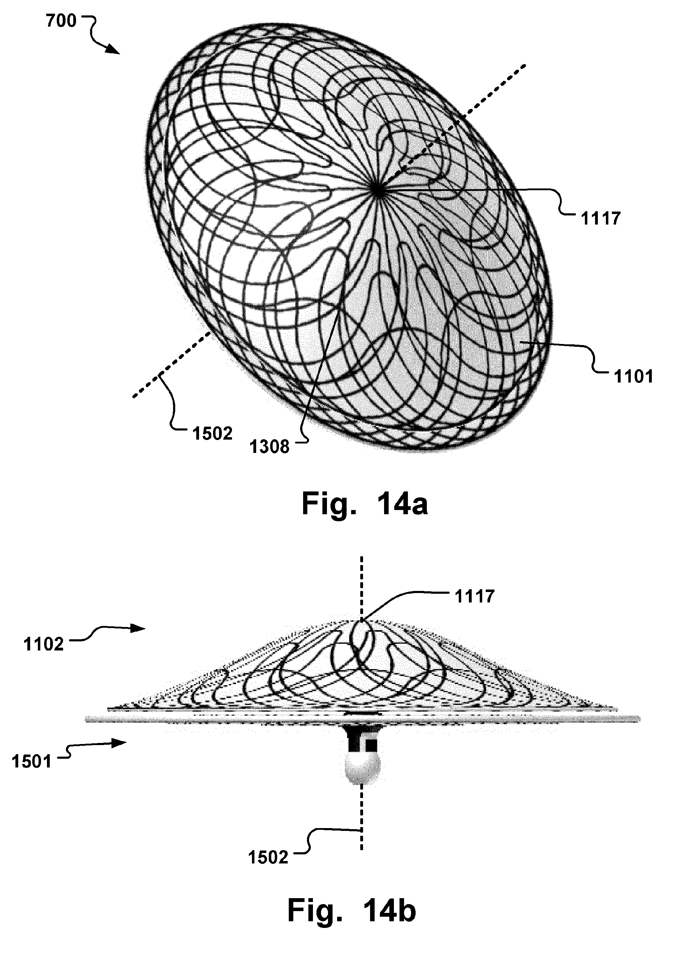

Figs. 14a-b, 15a-b, and 17, shows other embodiments of devices that benefit from the flexible braiding pattern. Fig. 9 is a perspective view of the device 200 in Figs. 7 and 8.

Returning to Fig. 8, at least one of the loop strands is displaced from the centre point by a centre distance 1109 (1110, 1210, 1310, cf. Figs. 10-12), and the apex points lie at a distance from the periphery 1113 of the expanded diameter portion that corresponds substantially to the radius of the expanded diameter portion minus the centre distance. The centre distance may vary, such as from 1/4th to 3/4th of the radius, to allow for different degrees of flexibility. lf the loop strands return to the proximal end at a larger distance from the centre point 1117, the flexibility may increase due to less wires at the expanded diameter portion 102 that exert a restraining force.

The loop strands form loops 1103, 1104, 1204, 1304 that may have a curved shape being concave radially outwards from the apex point 1107, 1108, 1208, 1308, to the periphery 1113. The curved shape may extend in a plane being substantially perpendicular to the longitudinal axis 104 of the device, at least in the relaxed state of the device. Thereby the curved loop strands may extend substantially in the plane spanned by the expanded diameter portion. The curved loop strands thereby help to provide the structural framework of the braid at the expanded diameter portion, that assist in holding the device in place at the target site, while allowing increased flexibility as they do not extend across the whole expanded diameter portion, e.g. in particular not across the centre. Of 30 wires, 20 may be looped back, and 10 may extend across the centre.

Further of embodiments will now be discussed having different braiding patterns for allowing increased flexibility of the expanded diameter portion. The expanded diameter portion is frequently referred to as just the distal end of the device.

Fig. 10 shows a braided implantable occlusion device 300 according to an embodiment of the invention. The device 300 comprises a mesh or braiding 101 of at least one thread. The braiding 101 may be formed from one thread or several. The device 300, or more particularly the braiding 101, has a collapsed state and an expanded state. Fig. 10 depicts an end of the device 300, which in this case is a distal end 1102. ln other embodiments (not shown) a proximal end of the device 300, or any other part of the device 300, may have the same features as the distal end 1102. Hence any part of the braiding 101 forming the device may have the features described in the following, with the associated advantages. The distal end 1102 is comprised of the braiding 101.

The braiding 101 may be made of a material suitable for implanting in a human or animal body, and suitable for being formed in a heat treatment procedure to a desired shape in an expanded state and also in the collapsed state. For example NiTinol may be used as a material for the device 300. However, suitable materials for embodiments of the braiding are various and include shape memory materials, metal, superelastic alloys (such as NiTinol), or polymers, such as degradable polymers.

The distal end 1102 comprises loops 1103, 1104, formed by loop strands 1105, 1106, of the at least one thread. In at least the expanded state each loop strand 1103, 1104, has a curved shape and extends away from a centre point 1117 of the distal end 1102. Thus, each of the loop strands 1105, 1106 has an apex point 1107, 1108, corresponding to the turning point of the curved shape and to the point of each of the loop strands that are closest to the centre point 1117.

In Fig. 10 the loops 1103, 1104, are U-shaped but may have other shapes of the open curvature, e.g. elliptical, half circular, or W-shaped. The curved shape of the loop strands 1103, 1104 extending away from the centre point 1117 should be construed as the opening of the U-shaped curve points radially outwards from the centre point 1117.

At least one of the loop strands 1105, 1106, is displaced from the centre point 1117 by a centre distance 1109, 1110, such that the location of the apex point 1107, 1108 is different from the centre point 1117.

The centre distance 1109, 1110, between the apex 1107, 1108, and the centre point may vary, and is preferably less than half the diameter (A) of the device 300, or less than half the cross-section at the location of the apex point in case the device 300 is non-circular.

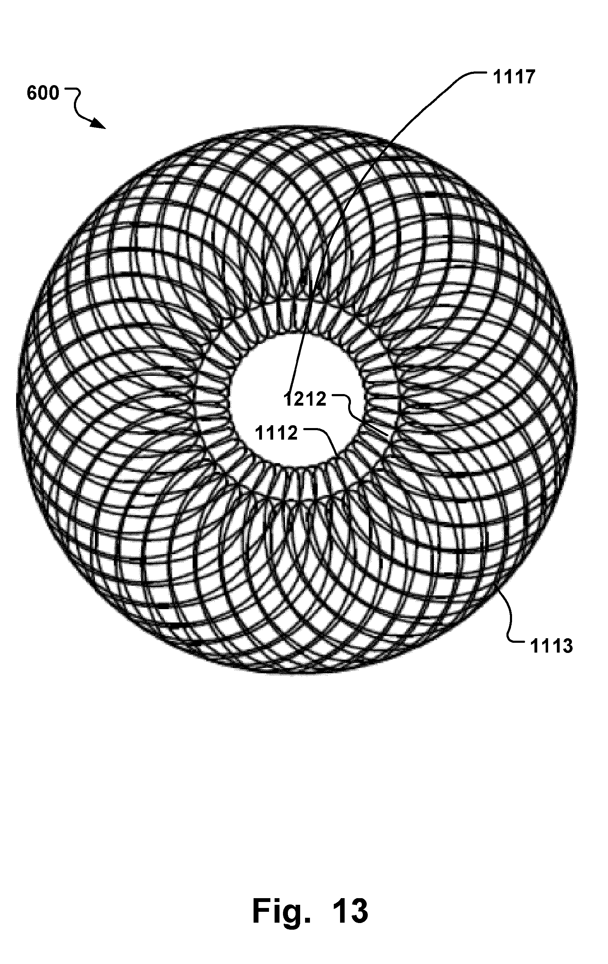

By having a displacement of at least one of the loop strands 1105, 1106, from the centre point 1117 the device 300 may exhibit a smaller cross-section or diameter in the collapsed state of the device 300, as less strands are present at the tip or centre point 1117 of the device. At the same time by having the apex point 1107, 1108, at a distance from the periphery 1113 of the distal end 1102 stability of the device 300 is maintained. I.e. a partly closed distal end 1102 is obtained even if no centre strands 1115 extend across the centre point 1117, as shown in Fig. 13. Having a partly closed distal end 1102, i.e. where the apex points 1107, 1108, of the loop strands are positioned between the centre 1105 and the periphery 1113 of the distal end 1102, may also facilitate occlusion by allowing for easier fixing of a membrane (not shown) in the braiding 101 of the distal end 1102 due to a part of the braiding 101 of the distal portion 1102 extending in the radial direction. The braiding 101 extending in the radial direction at the distal end 1102 may also improve the occlusion ability itself without the need for additional elements. As shown in Figs 2-6, 8, the apex points 1107, 1108, may be displaced from the centre 1105 by different distances. The flexibility of the device 101 may thereby be improved while maintaining structural rigidity.

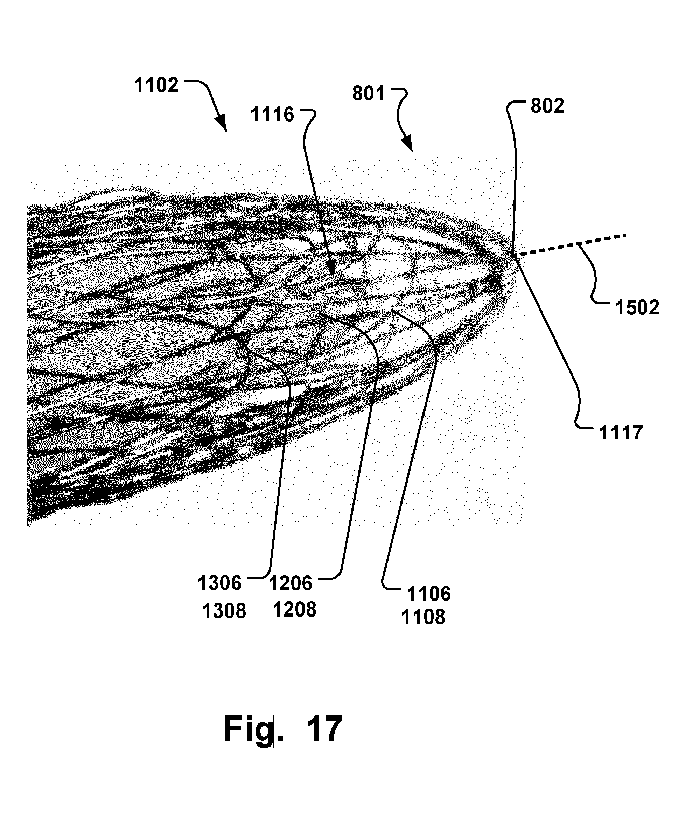

lf the device 300 is stretched substantially along a longitudinal axis 1502 passing through the centre point 1117, see Fig. 17, the centre point 1117 will correspond substantially to the tip of the device 300.

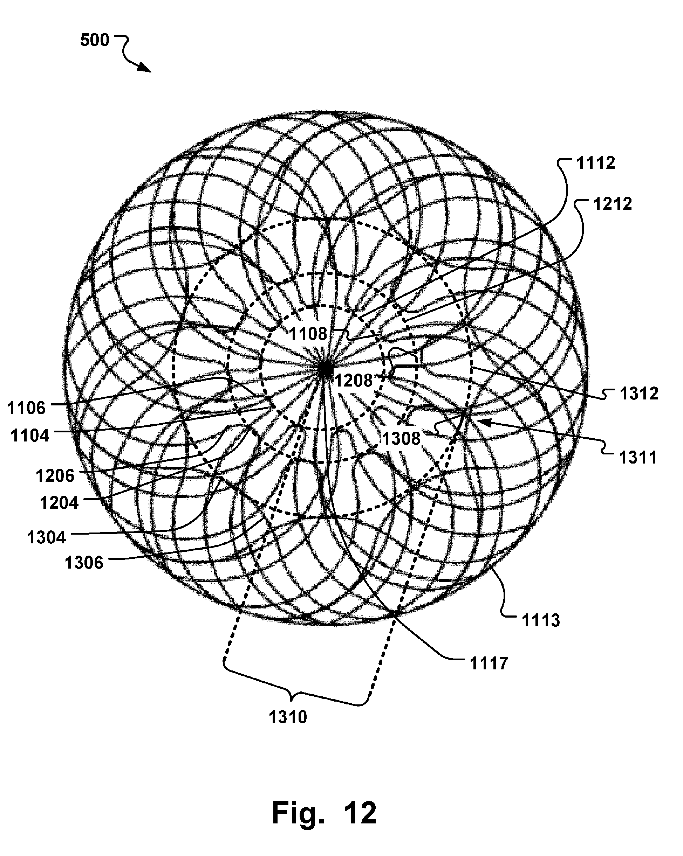

The device 100-800, is preferably collapsed by stretching. The device 100-800, may also be collapsed by compression. Hence, as the loop strands 1105, 1106, and further loop strands 1206, 1306, according to embodiments in Fig. 12, and Fig. 17, are displaced from the centre point 1117 less strands will be present at the tip 1801 which may reduce the cross-section of the tip 1801.

The apex point 1107, 1108, 1208, 1308, of each of the loop strands may be displaced from the centre point 1117 along a longitudinal axis 1502 when the device is in the collapsed state. The centre point 1117 may correspond to a distal tip 1802 when the device 100-800, is in the collapsed state, as shown in Fig. 17.

The braiding of the distal end 1102 may comprise a distal surface 1116 of the at least one thread. The distal surface 1116 extends from the apex point 1107, 1108, 1208, 1308, of each of said loop strands to the centre point 1117. The distal surface 1116 may comprise the loop strands 1105, 1106, 1206, 1306, or centre strands 1115. ln case the device 100-800, is in the collapsed state the distal surface 1116 may extend from the apex point 1107, 1108, 1208, 1308, of each of said loop strands to the distal tip 1802 of the device, as shown in Fig. 17. Hence, the braiding 101 in which the apex points 1107, 1108, 1208, 1308, is confined, may be continuous from these apex points to the centre point 1117. As shown in Fig. 13 the distal end 1102 of the device 600 may also be open. The centre strands 1115 improves the stability of the device, and/or occlusion effectiveness, while the flexibility and small cross-section is maintained in the collapsed state due to the loop strands being displaced from the centre point 1117.

By having a plurality of loop strands displaced from the centre point 1117 by a plurality of centre distances 1109, 1110, and further displacement by centre distances 1206, 1306, according to embodiments in Fig. 12, and Fig. 17, a larger portion of the distal end 1102 may exhibit a smaller cross-section in the collapsed state of the device 300. Further, the cross-section of the entire device 300 may be reduced by the displacement. Fig. 17 may be illustrative of a device 300 in both the collapsed state and in the expanded state. Hence, the cross-section may be reduced in the expanded state as well. By provision of a smaller cross-section the device 300 in the collapsed state the device 300 may be delivered to a target site in a patient through a delivery device with a reduced cross-section, which may lead to an easier delivery procedure or manipulation of the delivery device in the patient.

Further thanks to the displacement of the loop strands 1105, 1106, 1206, 1306, from the centre point 1117 the amount of force required to compress the device from the expanded state, as illustrated in Fig. 10-13, to the collapsed state, as illustrated in Fig. 17, is reduced. This is thanks to the fact that the loop strands 1105, 1106, 1206, 1306, do not cross the centre point 1117. Thus, the amount of threads that must be bent at the centre point 1117 when compressing the device 300 is reduced. Each thread crossing the centre point 1117 or a region close to the centre point 1117 that is subjected to substantial deformation when compressing the device 300 to the collapsed state has a certain amount of structural integrity and an associated force that must be exceeded in order to deform the thread. By having several loop strands 1105, 1106, 1206, 1306 displaced from the region subjected to the most of the deformation, e.g. the centre point 1117 or tip 1801, the force required for deformation is thus substantially reduced. A more flexible braided device 100-800 is thus obtained, which for example can be more easily retracted into a catheter sheath and which exerts less frictional force on the walls of the catheter thereby increasing the ease of operation of the device 100-800 in the catheter, for example during push and pull motion.

ln Fig. 10 the device 300 comprises a group 1111 of a plurality of loop strands 1105, 1106, that are displaced from the centre point 1117 such that the apex points 1107, 1108, of the group 1111 lie on an imaginary circle 1112 enclosing the centre point 1117. The apex points 1107, 1108 also lie at a distance from a periphery 1113 of the distal end 1102.

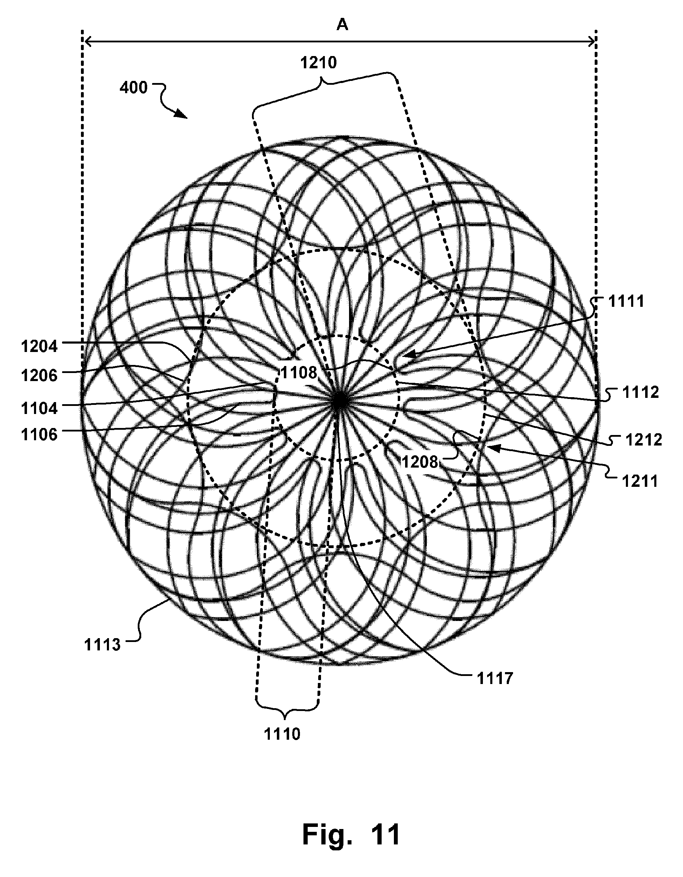

The stability of the device is thereby improved as compared to the case when the wires turn at the periphery of the braiding, as with the distal end of a device of a tubular braiding. It is also easier to manufacture the device according to the invention compared to such tubular braidings. As illustrated in Figs. 10-13 the distance the apex points lies from the periphery 1113 may vary from less than half of the radius, e.g. at a fourth of the radius, to more than half the radius, e.g. three fourths of the radius. Similarly, Fig. 11-13 shown devices 400-600 comprising at least one group 1111, 1211, 1311 of a plurality of loop strands 1105, 1106, 1206, 1306, that are displaced from the centre point 1117 such that the apex points 1107, 1108, 1208, 1308, of the at least one group lie on at least one imaginary circle 1112, 1212, 1312 enclosing the centre point 1117. The apex points 1107, 1108, 1208, 1308, also lie at a distance from a periphery 1113 of the distal end 1102. The radius of the at least one imaginary circle 1112, 1212, 1312 corresponds to the centre distances 1109, 1110, 1210, 1310. An increased radius may provide a more flexible device 100-600 with less force required for compression of the device 100-600 from its expanded state to its collapsed state.

The at least one imaginary circle 1112, 1212, 1312 may have its circle centre 1114 corresponding to the location of the centre point 1117. Thereby, the at least one group 1111, 1211, 1311 of the plurality of loop strands 1105, 1106, 1206, 1306, are displaced concentrically from the centre point 1117. Alternatively, the device 100-600 may have an asymmetrical position of the at least one imaginary circle 1112, 1212, 1312 with respect to the centre point 1117. Optionally, imaginary circles 1112, 1212, 1312 may be equidistantly distributed from each other in radial direction of the distal end 1102.

In Fig. 11 the loop strands 1106, 1206, comprise two groups 1111, 1211, of pluralities of loop strands. The first group 1111 and the second group 1211 of the plurality of loop strands have a first and second plurality of apex points 1108, 1208, respectively. The first and second plurality of loop strands are displaced from the centre point 1117 such that the first plurality of apex points 1108 lies on the periphery of an imaginary circle 1112 having a first radius 1110 and the second plurality of apex points 1208 lies on the periphery of an imaginary circle 1212 having a second radius 1210 which different from the first radius 1110. The first and second radius 1110, 1210, are less than the diameter (A) of the distal end 1102.

ln Fig. 12 a third group 1311 of the plurality of loop strands has a third plurality of apex points 1308 that lies on the periphery of an imaginary circle 1312 having a third radius 1310 different from the first and second radius 1110, 1210.

The first, second, and third groups 1111, 1211, 1311, of the plurality of apex points 1108, 1208, 1308, may lie concentrically with respect to the centre point 1117.

Each group 1111, 1211, 1311, of the pluralities of loop strands may be formed by a plurality of threads respectively, or by a single thread. The braiding 101 may comprise any number of threads.

The distal end 1102 may be closed, as shown in Fig. 10-12, preferably by a plurality of centre stands 1115 of the braiding 102 crossing each other at the centre point 1117. The distal end 1102 may also be open, as shown in Fig. 13. An open distal end 1102 may be advantageous in some applications.

The amount of the centre strands 1115 may be varied. The flexibility of the device 100-800 may be adjusted by varying the amount of centre strands 1115, hence providing customization of the device 100-800 to various applications. Fewer centre strands 1115 may decrease the force required for compressing the device 100-800 from the expanded state to the collapsed state, hence increasing the flexibility. The ratio between the amount of the centre strands 1115 and the loop strands may be set to a defined value. In Fig. 10 and Fig. 11 50% of the threads are looped back, i.e. are comprised of loop strands 1105, 1106, 1206, 1306. ln Fig. 11 75% of the threads are looped back, and in Fig. 13 100% of the threads are looped back. By varying the amount of loop strands the flow through the device may also be optimized. More or less dense loop strands or more strands crossing the centre may increase the maximum flow throughput of the device.

The device may comprise biocompatible fibres or patches of for example of PET that support sealing of the blood flow through the device.

Each of the apex points 1107, 1108, 1208, 1308 may be equally spaced apart around the peripheries of the at least one imaginary circle 1112, 1212, 1312.

The distal end 1102 may have any shape such as a circular disc shape, and/or spherical shape, and/or elongate shape.

Fig. 14a shows a perspective view of a device 700 having a braiding 101 according to the embodiment in Fig. 12. Fig. 14b show a side view of the device 700 in Fig. 14a according to an embodiment. Figs. 14a-b may represent a PFO occluder with two double layer discs. The braiding 101 of the device 700 may comprise loop strands according to any of the devices 100-600, e.g. one, two, or more groups of loop strands being displaced from the centre point 1117 at different distances.

As shown in Figs. 14a-b, a longitudinal axis 1502 extends between centre points of the distal and proximal ends the device 700. The occlusion device 700 has rotational symmetry around the longitudinal axis 1502, and the centre point 1117 coincide with the intersection of the longitudinal axis 1502 with the distal end 1102 of the device 700, which may also be the case for the devices 100-600.

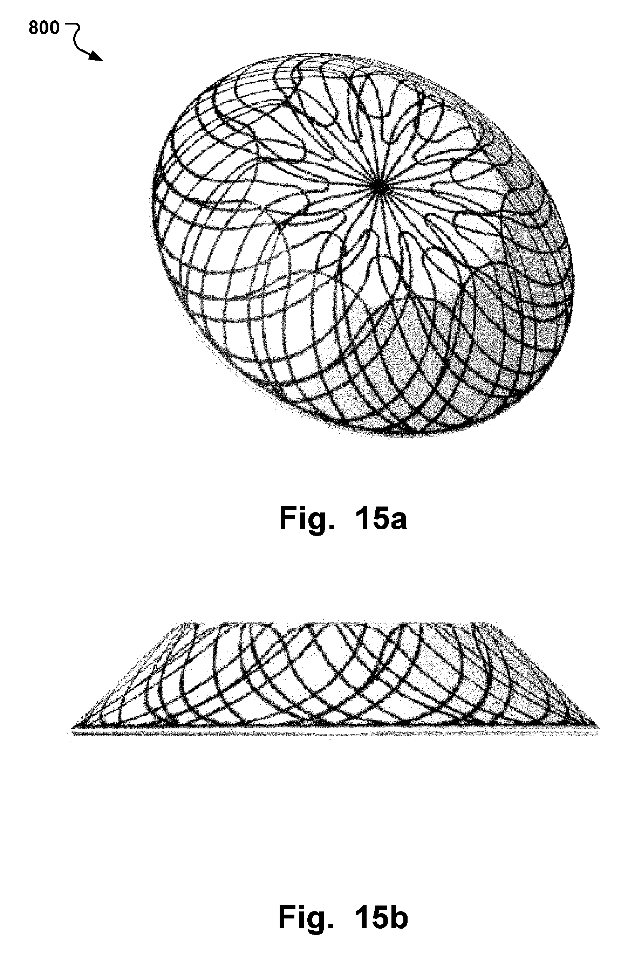

Figs. 15a shows a perspective view of a device 800 having a braiding 101 according to the embodiment in Fig. 12. Fig. 15b show a side view of the device 800 in Fig. 15a according to an embodiment. Figs. 15a-b may represent an ASD occluder with two double layer discs. The braiding 101 of the device 800 may comprise loop strands according to any of the devices 100-600.

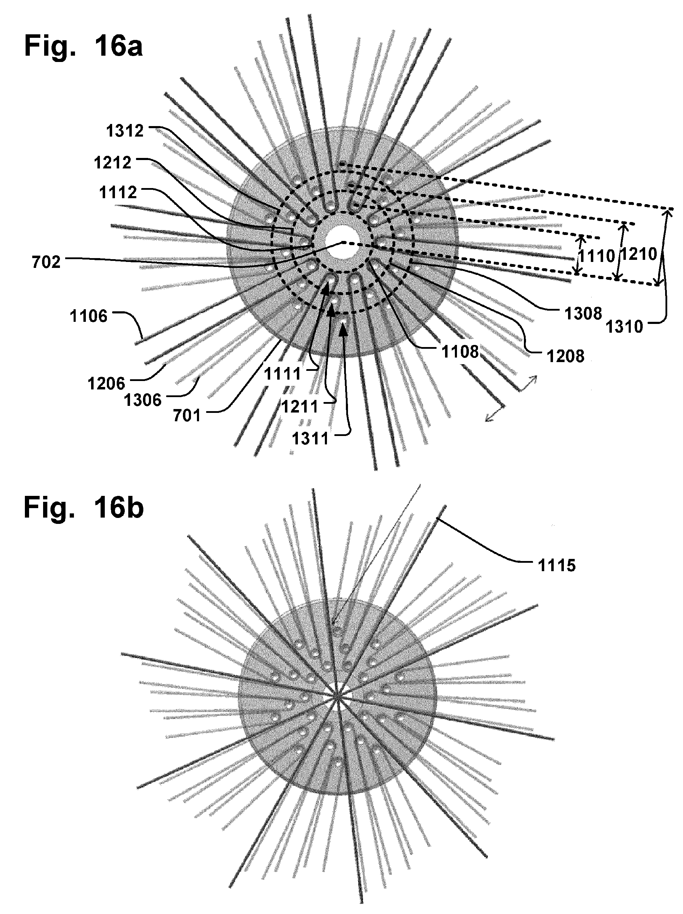

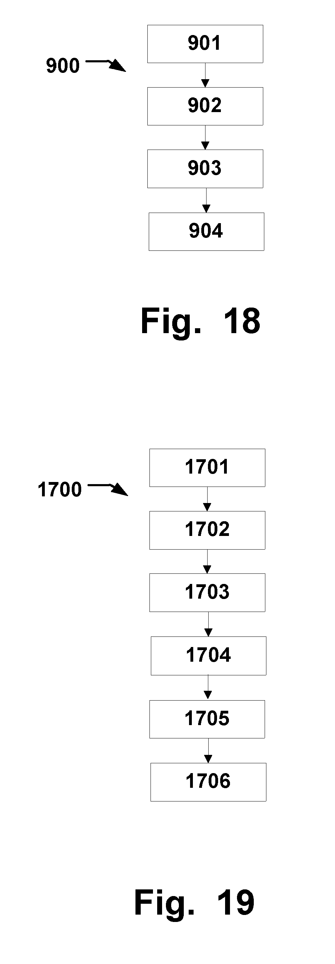

Figs. 16a-b and Fig. 18 illustrates a method 900 of manufacturing a medical implantable occlusion device 100-600 of a braiding 101 of at least one thread. The method 900 comprises forming 901 loops by loop strands 1106, 1206, 1306 of the at least one thread by an annular braiding tool 701 having a centre point 702. Each loop strand 1106, 1206, 1306 has a curved shape and extends away from the centre point 702 of the braiding tool 701. The apex point 1108, 1208, 1308, of each of the loop strands 1106, 1206, 1306, corresponds to the turning point of the curved shape and to the point of each of the loop strands 1106, 1206, 1306, being arranged closest to said centre point 702. At least one of the loop strands 1106, 1206, 1306, is displaced from the centre point 702 by a centre distance 1110, 1210, 1310, such that the location of the apex point 1108, 1208, 1308, is different from the centre point 702. As is shown in Fig. 7a-b the apex points 1108, 1208, 1308, lie at a distance from the peripheral edge of the braiding tool 701. A partially closed braiding 101, as discussed above, with returning loops may thereby be manufactured with improved stability over a mere tubular braiding, while maintaining flexibility. An improved occlusion ability is also provided, e.g. by the braiding 101 extending in the radial direction of the distal end 1102, or by easier incorporation of a membrane (not shown) in the braiding. As shown in Fig. 16a-b the apex points 1108, 1208, 1308, lie at different distances from the periphery of the braiding tool 701 or from the centre point 702. A braiding 101 with the loop strands displaced from the centre 1105 of the braiding 101 by different distances may thereby be manufactured. A device 100-800, having such braiding 101 has the advantages as described above.

The method 900 comprises forming 902 at least one group 1111, 1211, 1311, of a plurality of loop strands being displaced from the centre point 702 such that the apex points 1108, 1208, 1308, of the at least one group lie on at least one imaginary circle 1112, 1212, 1312, enclosing the centre point 1702.

The loops may be formed without crossing the loop strands 1106, 1206, 1306, with each other.

The method 900 may comprise forming 903 a closed end of the braiding by crossing 904 the centre point 702 with a plurality of centre strands 1115.

Fig. 19 illustrates a method 1700 of manufacturing a medical implantable occlusion device of a braiding 101 of at least one thread, comprising forming 1701 loop strands 1105, 1106, 1206, 1306, of the at least one thread at a distal end of the device, returning to the proximal end, whereby an apex point 1107, 1108, 1208, 1308 of each of the loop strands corresponds to the turning point of the loop strands and to the point of each of the loop strands being arranged closest to a centre point 1117 of the distal end, and
forming 501, 1702, the braiding in a first heat setting step to a first shape 405 comprising a tubular member 103 at the proximal end and an expanded diameter portion 102 at the distal end, wherein said apex points are positioned intermediate between a centre point 1117 of the expanded diameter portion and a periphery 1113 of the expanded diameter portion.

The device is that of the first aspect of the invention. Particularly advantageous anchoring and occlusion may be achieved while maintaining the aforementioned flexibility of the device. The shunt is for instance a Patent Ductus Arteriosus (PDA), Arterial Venous Fistula (AVF), Arterial Venous Malformation (AVM), or a Para-Valvular Leak (PVL).

The present invention has been described above with reference to specific embodiments. However, other embodiments than the above described are equally possible within the scope of the invention. The different features and steps of the invention may be combined in other combinations than those described. The scope of the invention is only limited by the appended patent claims.

More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. A medical implantable occlusion device (100-800) comprising a braiding (101) of at least one thread, said braiding comprising
an expanded diameter portion (102) at a distal end of said device,
a tubular member (103) at a proximal end of said device extending along a longitudinal axis (104),
wherein said expanded diameter portion comprises loops (1103, 1104, 1204, 1304), formed by loop strands (1105, 1106, 1206, 1306) of said at least one thread, returning to said proximal end,
whereby an apex point (1107, 1108, 1208, 1308) of each of said loop strands corresponds to the turning point of said loop strands and to the point of each of said loop strands being arranged closest to a centre point (1117) of said expanded diameter portion, and
wherein said apex points are positioned intermediate between said centre point (1117) and a periphery (1113) of said expanded diameter portion.

2. Occlusion device according to claim 1, wherein at least one of said loop strands is displaced from said centre point by a centre distance (1109, 1110, 1210, 1310), and wherein said apex points lie at a distance from said periphery (1113) of said expanded diameter portion that corresponds substantially to the radius of said expanded diameter portion minus said centre distance.

3. Occlusion device according to claims 1 or 2, wherein said loop strands form loops (1103, 1104, 1204, 1304), that each has a curved shape being concave radially outwards from said apex point to said periphery.

4. Occlusion device according to any of claims 1-3, wherein at least one group (1111, 1211, 1311) of a plurality of loop strands are displaced from said centre point such that said apex points of said at least one group lie on at least one imaginary circle (1112, 1212, 1312) enclosing said centre point, and/or wherein said distal end is closed, preferably by a plurality of centre strands (1115) of said braiding crossing each other at said centre point.

5. Occlusion device according to any of claims 1-4, wherein said distal end is closed, preferably by a plurality of centre strands (1115) of said braiding crossing each other at said centre point wherein the ratio between the amount of said centre strands and said loop strands is set to a defined value.

6. Occlusion device according to any of claims 1-5, said braiding comprises a stem (108) having a first end (109) connected to said expanded diameter portion and a second end (110) connected to said tubular member, wherein said expanded diameter portion is freely pivotable in relation to said tubular member via said stem.

7. Occlusion device according to claim 6, wherein said stem in a relaxed state of said braiding is partly sunk into said tubular member.

8. Occlusion device according to claim 6 or 7, wherein said braiding has an unloaded relaxed state and a stretched state,
said tubular member has a distal region (105) towards said expanded diameter portion and an opposite proximal region (106),
wherein a distal endpoint of said distal region closest to said expanded diameter portion has a distal rim (107) in a plane substantially orthogonal to said longitudinal axis,
wherein said stem in said relaxed state is partly sunk into said tubular member beneath said distal rim whereby said second end of said stem is displaced from said distal rim by a first offset (111) along said longitudinal axis.

9. Occlusion device according to any of claims 1-8, wherein said proximal region comprises a connecting member (113) for a delivery device, wherein ends of said at least one thread are fixed to said connecting member, and wherein said expanded diameter portion comprises returning loops of said at least one thread whereby opposite ends of said at least one thread forming said expanded diameter portion are fixed to said connecting member.

10. Occlusion device according to claim 8, wherein the distance between said distal rim and said expanded diameter portion is substantially smaller than the length of said stem.

11. Occlusion device according to any of claims 6-10, wherein said stem is pivotable with respect to said tubular member around said second end of said stem, and/or wherein said stem is concentric with respect to said tubular member, and/or wherein said tubular member is tapered towards said expanded diameter portion, and/or

12. Occlusion device according to any of claims 6-11, wherein said second end of said stem is connected to a distal surface (112) of said distal region, wherein said distal surface is sunk into said tubular member beneath said distal rim.

13. Occlusion device according to claim 12, wherein said distal surface is confined to a region of said braiding between said stem and said distal rim and having a diameter substantially equal to the diameter of said distal rim.

14. Occlusion device according to any of claims 1-13, wherein said tubular member and said expanded diameter portion are elastically stretchable relative each other along said longitudinal axis (104) such that a first offset (111) there between is variable in said relaxed state, such that said device is self-contracting, and/or wherein said expanded diameter portion is a disc shaped portion, and wherein the diameter of said expanded diameter portion is larger than a largest diameter of said tubular member.

15. A method (1700) of manufacturing a medical implantable occlusion device of a braiding (101) of at least one thread, comprising
forming (1701) loop strands (1105, 1106, 1206, 1306) of said at least one thread at a distal end of said device, returning to said proximal end, whereby an apex point (1107, 1108, 1208, 1308) of each of said loop strands corresponds to the turning point of said loop strands and to the point of each of said loop strands being arranged closest to a centre point (1117) of said distal end, and
forming (501, 1702) said braiding in a first heat setting step to a first shape (405) comprising a tubular member (103) at the proximal end and an expanded diameter portion (102) at the distal end, wherein said apex points are positioned intermediate between a centre point (1117) of said expanded diameter portion and a periphery (1113) of said expanded diameter portion.

16. Method of manufacturing a device according to claim 15, comprising
attaching (504, 1703) a secondary tool (401) to said braiding having said first shape, and forming (506, 1704) said braiding in a second heat setting step to a second shape (406) by said secondary tool, partly maintaining said first shape.

17. Method of manufacturing a device according to claim 15 or 16, wherein forming said second shape comprises
attaching (505, 1705) a secondary tool around a stem (108) of said braiding, wherein said stem is between and connects said tubular member and said expanded diameter portion,
moving (508, 1706) said secondary tool into said tubular member whereby said stem is partly sunk into said tubular member.
